# EUROPÄISCHE PATENTSCHRIFT

(11) **EP 3 872 186 B1**
(45) Veröffentlichungstag und Bekanntmachung des Hinweises auf die Patenterteilung: **03.04.2024**
(21) Anmeldenummer: 21153914.3
(22) Anmeldetag: 28.01.2021
(51) Int. Cl.: G01N 21/3563, C12Q 1/04, G01N 21/65, G01N 21/35

(54) **VERFAHREN ZUM SPEKTROMETRISCHEN CHARAKTERISIEREN VON MIKROORGANISMEN**
METHOD FOR SPECTROMETRIC CHARACTERIZATION OF MICROORGANISMS
MÉTHODE DE CARACTÉRISATION SPECTROMÉTRIQUE DE MICROORGANISMES

(30) Priorität: 27.02.2020 DE 102020105123
(43) Veröffentlichungstag der Anmeldung: 01.09.2021
(73) Patentinhaber: Bruker Daltonics GmbH & Co. KG, 28359 Bremen (DE)
(72) Erfinder: Mauder, Norman, 28211 Bremen (DE)
(74) Vertreter: Boßmeyer, Jens

(56) Entgegenhaltungen:
- EP-A1- 3 083 981
- EP-A1- 3 392 342
- WO-A1-2019/150408
- CN-A- 107 941 783
- NURLISA YUSUF ET AL: "In-vitro diagnosis of single and poly microbial species targeted for diabetic foot infection using e-nose technology", BMC BIOINFORMATICS, BIOMED CENTRAL , LONDON, GB, Bd. 16, Nr. 1, 14. Mai 2015 (2015-05-14), Seite 158, XP021220731, ISSN: 1471-2105, DOI: 10.1186/S12859-015-0601-5
- MORAIS CAMILO L ET AL: "Standardization of complex biologically derived spectrochemical datasets", NATURE PROTOCOLS, NATURE PUBLISHING GROUP, GB, Bd. 14, Nr. 5, 5. April 2019 (2019-04-05), Seiten 1546-1577, XP036769823, ISSN: 1754-2189, DOI: 10.1038/S41596-019-0150-X [gefunden am 2019-04-05]

## Beschreibung

### Gebiet der Erfindung

Die Erfindung betrifft Verfahren zum spektrometrischen Charakterisieren von Mikroorganismen, insbesondere unter Verwendung von Infrarot-spektrometrischen Methoden.

### Hintergrund der Erfindung

Der Stand der Technik wird im Folgenden mit Bezug auf einen speziellen Aspekt erläutert. Dies soll jedoch nicht als Einschränkung verstanden werden. Nützliche Fortentwicklungen und Änderungen vom aus dem Stand der Technik Bekannten können auch über den vergleichsweise engen Rahmen dieser Einleitung hinaus anwendbar sein und werden sich geübten Praktikern auf diesem Gebiet nach der Lektüre der nachfolgenden Offenbarung umstandslos erschließen.

Bei der spektrometrischen Charakterisierung von Mikroorganismen, z.B. Bakterien, Pilzen, Hefen, Algen oder Protozoen (auch Viren), können die spektrometrischen Messungen Organismus-externen Varianzen ausgesetzt sein, die den Inhalt der Spektren und folglich auch die Qualität der Analyse beeinflussen. Organismus-extern bedeutet, dass diese Varianzen nicht in der taxonomischen Einordnung des Mikroorganismus begründet sind.

Ein Beispiel kann bei der Infrarot-spektrometrischen Charakterisierung in den während der Messung herrschenden unterschiedlichen Feuchtebedingungen, denen die präparierten Proben im Messschacht des Infrarot-Spektrometers wegen der meist fehlenden Abschirmmaßnahmen unweigerlich ausgesetzt sind, ausgemacht werden. Der Feuchtegehalt der Proben wirkt sich unmittelbar auf die in einem Infrarot-Spektrum widergespiegelten Dreh- und Streckschwingungen aus. Beispielsweise ist der absolute Feuchtegehalt der Luft in warmen Jahreszeiten typischerweise höher als in kälteren Jahreszeiten (saisonale Abhängigkeit). Auch kann die Feuchte vom Ort der Messung abhängen. Man stelle sich ein mobiles Infrarot-Spektrometer vor, beispielsweise in einem Einsatzfahrzeug des Katastrophenschutzes, das sowohl an der Küste (tendenziell hohe Luftfeuchte) als auch im Hochland oder sogar Gebirge (tendenziell niedrigere Luftfeuchte) eingesetzt werden kann.

Problematisch wird diese Feuchtevarianz dann, wenn die eigentliche Charakterisierungsmessung unter Bedingungen ausgeführt wird, die sich von denen, die bei der Erfassung von Referenzdaten herrschen, deutlich unterscheiden. Bei der Charakterisierung von Mikroorganismen werden Referenzdaten häufig für die Ermittlung des phylogenetischen Taxons herangezogen, z.B. in der Hierarchie von oben nach unten Domäne, Phylum/Reich, Stamm, Klasse, Ordnung, Familie, Gattung und Art. Durch feuchteinduzierte Unterschiede kann die Verlässlichkeit der Charakterisierung leiden. Das Beispiel lässt sich von der Feuchte auf die im Messschacht herrschende Temperatur übertragen, weil die Temperatur zumindest indirekt die Fähigkeit der präparierten Proben zur Feuchtigkeitsaufnahme und damit die Schwingungseigenschaften der Moleküle beeinflusst.

In dem zuvor genannten Beispiel ließen sich instrumentelle Maßnahmen treffen, um die aufgeführten Probleme abzumildern. So könnte ein hermetisch versiegelbarer Messschacht für das Infrarot-Spektrometer konstruiert werden oder der Messschacht könnte kontinuierlich mit einem bezüglich Feuchte und Temperatur vorkonditionierten Gas gespült werden, so dass in ihm während der Messung konstante einheitliche Bedingungen aufrechterhalten werden. Diese Konstanz würde dafür sorgen, dass kein wesentlicher Unterschied zu den Bedingungen besteht, die während der Aufzeichnung von Referenzdaten herrschen. Allerdings hat sich diese hermetische Versiegelung bei den Spektrometer-Herstellern nicht durchgesetzt.

Es gibt vielfältige Berichte über die spektrometrische Charakterisierung von Mikroorganismen. Auszugsweise wird auf die folgenden Dokumente verwiesen:

US 2008/0132418 A1 erläutert ein Verfahren zum Charakterisieren eines Mikroorganismus für den Zweck der medizinischen Diagnose oder Nahrungsmittel- und Umweltkontrolle. Mindestens ein Spektralbild des Mikroorganismus mit mehreren Pixeln wird erhalten und ein oder mehrere Spektren werden aus dem Spektralbild mit mehreren Pixeln auf der Basis von vorbestimmten Spektraleigenschaften ausgewählt, wobei die ausgewählten Spektren Spektralinformationseigenschaften des Mikroorganismus umfassen. Für die Identifikation können die ausgewählten Spektren des Mikroorganismus mit Spektren von Bezugsmikroorganismen in einer Datenbank verglichen werden. Die Datenbank kann erstellt werden durch: Erhalten von mindestens einem Spektralbild mit mehreren Pixeln für jeden von einer Vielzahl von Bezugsmikroorganismen, wobei jedes Pixel ein Signal aufweist, das einem Spektrum eines Bezugsmikroorganismus entspricht; und Auswählen von Spektren aus den Spektralbildern mit mehreren Pixeln auf der Basis von vorbestimmten Spektraleigenschaften, um die Datenbank zu erstellen, wobei die Datenbank mindestens ein Spektrum für jeden der Bezugsmikroorganismen umfasst.

M. J. Gupta et al. (Transactions of the ASABE, Vol. 49(4), 2006, 1249-1255) berichten über die Identifizierung und Quantifizierung von vier lebensmittelgängigen Enterobakterien (*Escherichia coli* 026, *Salmonella typhimurium, Yersinia enterocolitica* und *Shigella boydii*) in vier verschiedenen Lebensmittelmatrices mit Hilfe von FTIR-Spektroskopie, aus den Infrarot-Spektren abgeleiteten Hauptkomponenten und Künstlichen Neuronalen Netzen (KNN). Die Klassifizierungsgenauigkeit der KNN zur Identifizierung und Quantifizierung wird mit 93,4% bzw. 95,1% angegeben. Die KNN wurden anhand eines unabhängigen Datensatzes validiert, der aus einem separat gezüchteten und vermehrten Enterobakterium gewonnen wurde. Die Genauigkeit der KNN wird für eine solche Validierung beim Nachweis von *Yersinia enterocolitica* zwischen 64% bis 100% angegeben. Herausforderungen bei der Filterung des Hintergrundrauschens in den Spektren werden hervorgehoben.

EP 2 174 116 B1 beschreibt ein Verfahren zum Typisieren oder Identifizieren eines Mikroorganismus unter Verwendung von Schwingungsspektroskopie. Eine schwingungsspektroskopische Analyse wird an einer Mikroorganismenprobe durchgeführt, bei der die durch bleichbare Komponenten in der Probe verursachte Signalvarianz im Wesentlichen aus den erhaltenen Schwingungsspektren eliminiert wird. Die bleichbaren Komponenten werden als solche identifiziert, die Schwingungsspektralbänder erzeugen, die beim Photobleichen eine Verringerung der Intensität zeigen. Dadurch sollen korrigierte schwingungsspektroskopische Informationen zum Zweck des Typisierens und Identifizierens bereitgestellt werden.

US 2002/138210 A1 offenbart ein Verfahren zum Kompensieren von Drift in Fingerabdruckspektren aufgrund von Umweltfaktor-Änderungen, umfassend: Kultivieren eines interessierenden Mikroorganismus und eines zweiten Mikroorganismus, der dem interessierenden Mikroorganismus vermutlich metabolisch ähnlich ist, unter einem ersten Satz von Umweltfaktoren; Messen eines Fingerabdruckspektrums des Mikroorganismus von Interesse, der unter dem ersten Satz von Umweltfaktoren kultiviert wurde, und eines Fingerabdruckspektrums des zweiten Mikroorganismus, der unter dem ersten Satz von Umweltfaktoren kultiviert wurde; Detektieren von Unterschieden zwischen dem Fingerabdruckspektrum des zweiten Mikroorganismus, der unter dem ersten Satz von Umweltfaktoren kultiviert wurde, und einem Fingerabdruckspektrum des zweiten Mikroorganismus, der unter einem zweiten Satz von Umweltfaktoren kultiviert wurde; und Verwenden der Unterschiede zwischen den Fingerabdruckspektren des zweiten Mikroorganismus, der unter den zwei Sätzen von Umweltfaktoren kultiviert wurde, um das Fingerabdruckspektrum des Mikroorganismus von Interesse, der unter dem ersten Satz von Umweltfaktoren kultiviert wurde, in ein erwartetes Fingerabdruckspektrum für den Mikroorganismus von Interesse unter dem zweiten Satz von Umweltfaktoren zu transformieren.

EP 1 319 176 B1 beschreibt ein Verfahren zum Charakterisieren von Spektrometer-Instrumenten gemäß der instrumentellen Variation, welche zwischen Instrumenten vorliegt, und/oder der Variation über die Zeit innerhalb des gleichen Instruments, umfassend die Schritte: Vorsehen einer Mehrzahl von Spektren bekannter Standards von mindestens einem Spektrometer-Instrument; Klassifizieren des mindestens einen Spektrometer-Instruments in mindestens einen von einer Mehrzahl vordefinierter Cluster auf der Basis von spektralen Merkmalen, welche aus dem mindestens einen Spektrum extrahiert wurden; und Vorsehen von mindestens einem Kalibrationsmodell für jeden der vordefinierten Cluster, wobei jedes Kalibrationsmodell die instrumentelle Variation von Instrumenten, die in den jeweiligen Clustern klassifiziert wurden, kompensiert.

Die Studie von Yusuf et al. BMC Bioinformatics (2015) 16:158 beschäftigt sich mit der In-vitro-Diagnose einzelner und mehrerer mikrobieller Spezies bei diabetischen Fußinfektionen mit Hilfe der e-nose-Technologie.

Die Patentveröffentlichung WO 2019/150408 A1 offenbart eine Methode zur Klassifizierung und Identifizierung von Mikroorganismen unter Verwendung der Fourier Transform-Infrarotspektroskopie mit abgeschwächter Totalreflexion (FTIR-ATR).

Es besteht daher ein Bedarf, spektrometrische Charakterisierungen von Mikroorganismen auch in Gegenwart von Varianzeinflüssen während der Messung robuster und verlässlicher zu machen. Weitere von der Erfindung zu lösende Aufgaben ergeben sich für den Fachmann ohne weiteres bei der Lektüre der nachfolgenden Offenbarung.

### Zusammenfassung der Erfindung

Die Erfindung betrifft Verfahren zum spektrometrischen Charakterisieren von Mikroorganismen, insbesondere unter Verwendung von Infrarot-spektrometrischen Methoden.

Zur Klassifizierung und Identifizierung von Mikroorganismen, z.B. Bakterien, Pilze, Hefen, Algen oder Protozoen (auch Viren), gibt es viele verschiedene Analysetechniken, aber viele davon fußen auf spezifischen biochemischen Stoffen, die extrahiert, modifiziert und nachgewiesen werden müssen. Am häufigsten werden Testsysteme eingesetzt, die die Eigenschaften und Enzymaktivitäten von Bakterien beschreiben, z.B. Gallenlöslichkeit, Katalase, Koagulase, DNase, Motilität, Toxine, Optochin, Streptex, Oxidase, Gram-Färbung, Ziehl-Neelsen-Färbung, Sporen, Kapseln, Fermentationsfähigkeiten usw. Andere häufig angewandte Methoden sind zelluläre Fettsäure-Analyse, Proteinprofilierung, Polymerase-Kettenreaktion (PCR) und 16S rRNA-Gen-Sequenzierung, Gesamt-DNA G+C Inhalt, hochauflösende Gas/Flüssigkeits-Chromatographie und Mikroskopie, um die Zellmorphologie aufgrund unterschiedlicher Form und Erscheinung zu bestimmen. Alle zuvor genannten Methoden haben gemeinsam, dass sie nur einen bestimmten Marker der Bakterien zur Klassifizierung verwenden. Im Gegensatz dazu erfasst die Infrarot-Spektrometrie alle in einer Bakterienzelle vorhandenen Biomoleküle gleichzeitig, da fast jedes Biomolekül Infrarotstrahlung absorbiert. Die aus der Wechselwirkung zwischen Infrarotstrahlung und Biomolekül resultierende spektrale Information umfasst alle zellulären Bestandteile und kann daher als nahezu vollständige phänotypische Beschreibung der analysierten Bakterien verwendet werden.

Ein Prüfmikroorganismus wird bereitgestellt, dessen Identität auf einer ersten taxonomischen Ebene bekannt ist. Die Identität des Prüfmikroorganismus auf der ersten taxonomischen Ebene kann insbesondere vorab durch wenigstens eines der folgenden Verfahren ermittelt worden sein: (i) Massenspektrometrie, (ii) Infrarot-Spektrometrie, (iii) Wachstum auf selektiven Medien ("bunte Reihe") und (iv) Gensequenzanalysen. Ein Beispiel für die Ermittlung des Vorwissens ist in DE 10 2013 022 016 B4 zu finden, wo insbesondere eine massenspektrometrische Bestimmung der Art eines Mikroorganismus durch eine Infrarot-spektrometrische Bestimmung der Unterart ergänzt wird. Das Bereitstellen kann weiterhin die Isolierung des Prüfmikroorganismus aus einem Habitat, z.B. einer biologischen und/oder chemischen Matrix, umfassen. Vorzugsweise beinhaltet das Isolieren des Prüfmikroorganismus das Entfernen der Matrix, z.B. durch Waschen, Filtern, Abzentrifugieren, Eindampfen, Absaugen, Sedimentieren und/oder sonstiges Abscheiden. Das Bereitstellen des Prüfmikroorganismus kann überdies einen Vermehrungsschritt beinhalten, z.B. das Bebrüten in einer Nährlösung oder auf einem flächigen Nährmedium wie Agar.

Der Prüfmikroorganismus wird erfindungsgemäß sterilisiert. Das Sterilisieren kann ein Exponieren des Prüfmikroorganismus gegenüber einer Stoffwechsel-hemmenden Flüssigkeit, z.B. einem Alkohol wie Ethanol oder Iso-Propanol oder einer Säure wie Ameisensäure, und/oder Energieeinwirkung, z.B. mit Wärme oder hochenergetischer Strahlung (ggfs. ultraviolettes Licht), umfassen. Unter Sterilisieren wird insbesondere verstanden, dass der Mikroorganismus die Fähigkeit verliert, sich selbst unter für ihn günstigen Bedingungen zu vermehren, um die mit unbeabsichtigter/unkontrollierter Verbreitung einhergehenden biologischen Gefahren in einem Analyselabor zu vermeiden. Unter bestimmten Bedingungen kann es entbehrlich sein, den Prüfmikroorganismus zu sterilisieren, beispielsweise dann, wenn in einem Analyselabor der biologischen Sicherheitsstufe 2 oder höher gearbeitet wird, weil dort davon ausgegangen werden kann, dass hinreichend geschultes Fachpersonal zum Einsatz kommt.

Es werden spektrometrische Messdaten vom sterilisierten Prüfmikroorganismus unter Bedingungen aufgenommen, die den Einfluss wenigstens einer Varianzquelle zulassen, welche nicht in der taxonomischen Einordnung des Prüfmikroorganismus begründet ist. Die wenigstens eine Varianz kann atmosphärischen Ursprungs sein und insbesondere unterschiedliche Werte auf wenigstens einer der folgenden Skalen umfassen: Temperatur, Luftfeuchte, Druck und Kohlendioxid-Gehalt der Umgebungsluft. Eine Varianz atmosphärischen Ursprungs oder atmosphärische Varianz äußert sich in Änderungen der Bedingungen nahe einer und um eine für eine spektrometrische Messung präparierte Prüfmikroorganismen-Probe herum, üblicherweise präpariert auf einem Objektträger, z.B. einem Glasplättchen. Bevorzugt betrifft die wenigstens eine Varianz beeinflussbare Änderungen auf einer kontinuierlichen und endlichen Skala, insbesondere solche, die wenigstens einseitig begrenzt sind, z.B. der absolute Nullpunkt bei der Temperatur, oder eine Untergrenze und Obergrenze aufweisen, z.B. 0% bis 100% bei der relativen Feuchte.

Ein Klassifikator wird ausgewählt, der darauf trainiert ist, die Identität eines Mikroorganismus auf einer zweiten taxonomischen Ebene, die der ersten taxonomischen Ebene nachgeordnet ist, zu ermitteln, wobei mögliche Identitäten des Klassifikators auf der zweiten taxonomischen Ebene der bekannten Identität des Prüfmikroorganismus auf der ersten taxonomischen Ebene zugeordnet sind. Die erste taxonomische Ebene und die zweite taxonomische Ebene können unmittelbar benachbart sein, z.B. Art (Spezies) bzw. Unterart (Subspezies). Ferner kann die erste taxonomische Ebene der Art (Spezies) entsprechen und die zweite taxonomische Ebene kann verschiedene Varietäten umfassen, z.B. pathogene und nicht-pathogene Varietäten, resistente und empfindliche (suszeptible) Varietäten oder verschiedene Stämme der Art. Diese Varietäten/Stämme unterschiedlichen Typs können zum Zwecke der Charakterisierung zu Identitätsunterklassen zusammengefasst werden, z.B. alle pathogenen Serotypen/Stämme in eine erste Gruppe und alle nicht-pathogenen Serotypen/Stämme in eine andere, davon abgegrenzte Gruppe.

Mit Serotyp oder Serovar (Kurzform von *Serovarietas*) bezeichnet man Varietäten innerhalb von Subspezies von Bakterien, die mit serologischen Tests unterscheidbar sind: sie unterscheiden sich durch Antigene an der Oberfläche der Zellen und werden in der klassischen Mikrobiologie mittels spezifischer Antikörper identifiziert. Die taxonomische Hierarchie für Serotypen ist die folgende: Gattung => Art (Spezies) => Unterart (Subspezies, subsp.) => Serotyp, beispielsweise mit dem ergänzten binomischen Artnamen *Salmonella enterica* subsp. *enterica* serotyp Typhi, Kurzform *Salmonella* Typhi.

Ein Pathovar (von griechisch *pathos "*Leiden") ist ein Bakterienstamm oder eine Gruppe von Stämmen mit denselben Eigenschaften, die innerhalb der Art oder Unterart aufgrund ihrer Pathogenität von anderen Stämmen abgegrenzt werden können. Pathovare werden mit Dritt- oder Viertzusatz zum binomischen Artnamen gekennzeichnet. Das Bakterium *Xanthomonas axonopodis* beispielsweise, das Zitronenkrebs verursachen kann, hat verschiedene Pathovare mit unterschiedlichen Wirtsspezialisierungen: *X. axonopodis* pv. *citri* ist eins davon, die Abkürzung "pv." bedeutet "Pathovar". Auch die virulenten Stämme humanpathogener Erreger besitzen Pathovare, die hier aber durch Namensvorsätze gekennzeichnet werden. Das zumeist völlig harmlose Darmbakterium *Escherichia coli* weist zum Beispiel die höchst gefährlichen Pathovare enterohämorrhagische *E. coli* (EHEC), enteropathogene *E. coli* (EPEC), enterotoxinbildende *E. coli* (ETEC), enteroinvasive *E. coli* (EIEC), enteroaggregative *E. coli* (EAEC) sowie diffus adhärente *E. coli* (DAEC) auf. Die Pathovare können wiederum verschiedene Serotypen umfassen: so gibt es bei EHEC viele bekannte Serotypen, wobei etwa 60 Prozent aller identifizierten EHEC-Serotypen auf 0157, 0103, und O26 entfallen. Besonders gefährlich ist der Sero-Untertypus 0157/H7.

Im weiteren Sinne kann die Charakterisierung von Mikroorganismen auch Varietäten umfassen, die sich durch andere medizinisch relevante Merkmale unterscheiden, insbesondere durch das Resistenzverhalten gegenüber antimikrobiellen Mitteln wie Antibiotika (insbesondere Beta-Lactam-Antibiotika und Glykopeptid-Antibiotika) und Antimykotika, aber auch durch die Toxinbildung ("Toxivaren") oder durch die Empfänglichkeit für gleiche oder ähnliche Bakteriophagen ("Phagovaren"). Ganz allgemein spricht man von "Biovaren", wenn eine Auswahl von Mikroorganismen einer Spezies oder Subspezies biologische Gemeinsamkeiten besitzt. Ein Beispiel für eine antibiotika-resistente Varietät ist MRSA: Methicillin-resistenter *Staphylokokkus aureus.*

Der Begriff "Stamm" (genauer "Kulturstamm" oder "Abstammungslinie", englisch *strain*) beschreibt eine Population, die aus einem einzelnen Organismus heraus vermehrt wurde und in einer (häufig staatlichen) Sammelstelle für Mikroorganismenstämme aufbewahrt wird, wobei an die Namenskette mit Gattung, Art, Unterart und Varietät eine international normierte Stammbezeichnung angefügt wird. Die einzelnen Organismen eines Stammes sind genetisch identisch; verschiedene Stämme variieren leicht in ihrer genetischen Ausstattung.

Der Klassifikator wird auf die Messdaten angewendet, um die Identität des Prüfmikroorganismus auf der zweiten taxonomischen Ebene zu ermitteln. Der Klassifikator ist außerdem varianzkonditioniert, indem er erhalten ist durch Trainieren auf gezielt varianzbehafteten spektrometrischen Referenzdaten verschiedener bekannter Referenzmikroorganismen, welche die gleiche Identität auf der ersten taxonomischen Ebene wie der Prüfmikroorganismus aufweisen und verschiedene Identitäten auf der zweiten taxonomischen Ebene abdecken, wobei das Trainieren die Maßgabe umfasst, diejenigen spektralen Merkmale eines ersten Typs aus den Referenzdaten, die eine Unterscheidung der verschiedenen Identitäten auf der zweiten taxonomischen Ebene fördern, gegenüber denjenigen spektralen Merkmalen eines zweiten Typs aus den Referenzdaten, die von der gezielten Varianz beeinflusst sind, aufzugewichten, um den Varianzeinfluss bei der Charakterisierung des Prüfmikroorganismus auf der zweiten taxonomischen Ebene weitgehend oder vollständig auszublenden.

Offenbart wird ferner eine Anwendung eines bezüglich wenigstens einer Varianz konditionierten Klassifikators zur Unterscheidung von Identitäten eines Mikroorganismus auf einer vorbestimmten taxonomischen Ebene auf spektrometrische Messdaten, welche von einem sterilisierten Prüfmikroorganismus unter Bedingungen aufgenommen wurden, die den Einfluss der wenigstens einen Varianz zulassen, wobei die wenigstens eine Varianz nicht in der taxonomischen Einordnung des Mikroorganismus begründet ist.

Fachleute auf dem Gebiet werden erkennen, dass alle im Zusammenhang mit dem Verfahren offenbarten Ausführungen auch auf die Anwendung übertragbar sind.

### Kurze Beschreibung der Abbildungen

Zum besseren Verständnis der Erfindung wird auf die folgenden Abbildungen verwiesen. Die Elemente in den Abbildungen sind nicht unbedingt maßstabsgetreu dargestellt, sondern sollen in erster Linie die Prinzipien der Erfindung (größtenteils schematisch) veranschaulichen:

Abbildung 1A zeigt eine Hauptkomponentenanalyse in einem dreidimensionalen Raum aus Infrarot-Spektren verschiedener Serotypen (SV) von *Streptococcus pneumoniae* in Abhängigkeit einer variierenden relativen Feuchte (rH; *relative humidity*).

Abbildung 1B zeigt die Auswertung der gleichen Spektren wie in Abbildung 1A, allerdings mittels linearer Diskriminanzanalyse darauf ausgerichtet, den Einfluss der variierenden Feuchte in den spektralen Merkmalen zu beseitigen.

Abbildung 2 zeigt eine stark vereinfachte Messaufbauskizze eines Fourier Transformations-Infrarotspektrometers (FT-IR) in Transmission mit aufgenommenem Spektrum.

Abbildung 3 zeigt einen beispielhaften Ablauf der Präparation einer spektrometrischen Mikroorganismen-Probe vor der Messung (im Gegenuhrzeigersinn).

Abbildung 4 zeigt eine Hauptkomponentenanalyse in einem zweidimensionalen Raum aus Infrarot-Spektren verschiedener Serotypen (SV) von *Legionella pneumophila* in Abhängigkeit einer variierenden relativen Feuchte (rH; *relative humidity*), wobei die Serotypen nach Pathogenität in Gruppen zusammengefasst sind.

### Detaillierte Beschreibung

Die vorliegende Erfindung ist im Anspruch 1 definiert. Während die Erfindung anhand einer Anzahl von Ausführungsformen dargestellt und erläutert wurde, werden Fachleute auf dem Gebiet anerkennen, dass verschiedene Änderungen in Form und Detail daran vorgenommen werden können, ohne vom Umfang der in den beigefügten Patentansprüchen definierten technischen Lehre abzuweichen.

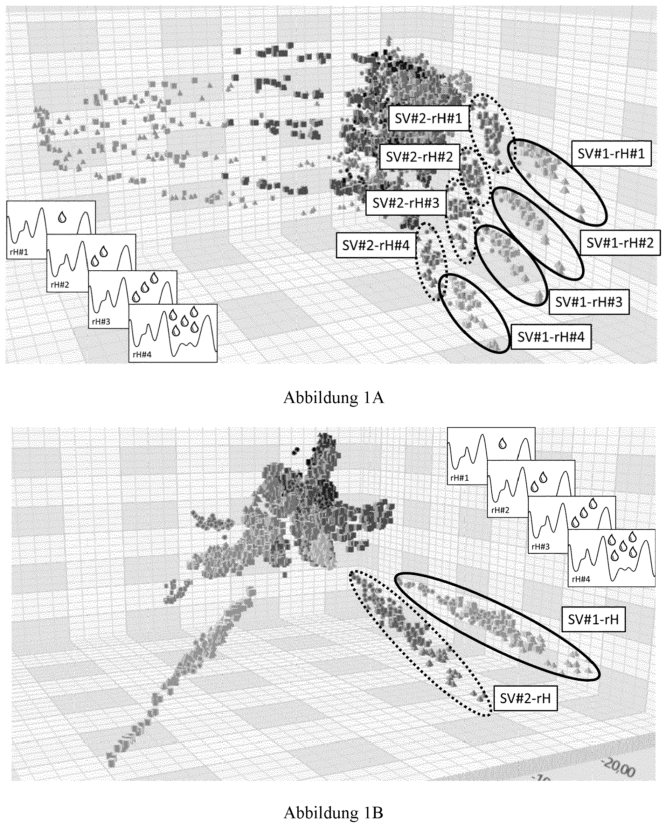

Abbildung 1A zeigt eine Hauptkomponentenanalyse von Infrarot-Spektren verschiedener Serotypen (SV) des Mikroorganismus *Streptococcus pneumoniae* in Abhängigkeit von vier verschiedenen Feuchteniveaus zwischen 10% und 80% relativer Feuchte (rH#1 bis rH#4), die im Messschacht des Infrarot-Spektrometers für die Messung gezielt eingestellt wurden. Wie zu erwarten, streuen die einzelnen Anhäufungen der Datenpunkte über die Serotypen, wobei in der Abbildung 1A die vier resultierenden Datenwolken zweier Beispiels-Serotypen der Übersichtlichkeit halber mit unterschiedlich strich-punktierten Ovalen hervorgehoben sind (SV#1 und SV#2). Den Messungen lagen jeweils mehrere Stämme/Isolate eines Serotyps zu Grunde, was sich in den langgestreckten Datenwolken bei den einzelnen Feuchteniveaus niederschlägt. Die unterschiedlichen Stämme/Isolate sind durch unterschiedliche Formen wie Pyramiden, Würfel und Kugeln kenntlich gemacht. Darüber hinaus ist die Trennung der Messungen vom gleichen Serotyp über die verschiedenen Feuchteniveaus klar erkennbar. Es zeigt sich ebenfalls überraschenderweise, dass die Feuchteabhängigkeit der spektralen Merkmale nicht dazu führt, dass Datenwolken verschiedener Serotypen in- und/oder durcheinanderlaufen. Stattdessen bleiben die einzelnen Datenwolken der verschiedenen Serotypen auch über verschiedene Feuchteniveaus getrennt ("stratifiziert") und können somit im Zuge einer Charakterisierung unterschieden werden.

Dieser Befund gab Anlass zu der Vermutung, dass sich diese Eigenschaft der beständigen Unterscheidbarkeit auch bei beträchtlicher Varianz (10% bis 80% auf der Feuchte-Skala) während einer spektrometrischen Messung dazu nutzen lässt, diese Varianz mittels fortgeschrittener Auswertemethoden, wie zum Beispiel Methoden des maschinellen Lernens, bei der Charakterisierung herauszurechnen, so dass aufwändige Umrüstungen der dafür verwendeten Spektrometer entbehrlich werden.

Die Spektren, die der Darstellung in Abbildung 1A zu Grunde liegen, wurden dementsprechend ein zweites Mal ausgewertet. Zur Anwendung kam eine lineare Diskriminanzanalyse, der vorgegeben wurde, diejenigen spektralen Merkmale, z.B. Hauptkomponenten, die die Unterscheidungskraft zwischen den einzelnen bekannten Serotypen maximieren, zu identifizieren und aufzugewichten, wohingegen diejenigen spektralen Merkmale, die von den Varianzen innerhalb der einzelnen Serotyp-Klassen beeinflusst werden, welche im Wesentlichen von den unterschiedlichen Stämmen (allerdings eine Organismus-interne Varianz) und Feuchteniveaus bei der Messung herrühren, so weit wie möglich ausgeblendet werden sollen.

Das Ergebnis ist in Abbildung 1B wiedergegeben. Wie zu erkennen ist, wurden die vier unterschiedlichen Feuchteniveaus, die sich in Abbildung 1A in einer Schicht aus vier deutlich voneinander getrennten Datenwolken pro Serotyp äußern, feuchteunabhängig in eine zusammenhängende, spindelförmige Datenwolke pro Serotyp zusammengeschrumpft, ohne dass sich die einzelnen Serotyp-Datenwolken überlagern oder durchdringen. Dieses Ergebnis kann dazu verwendet werden, einen varianzkonditionierten Klassifikator zu erstellen, der - wenn er auf ein neues Spektrum von *Streptococcus pneumoniae* mit noch unbekanntem Serotyp angewendet wird - die neu ermittelten spektralen Merkmale den bekannten Raumvolumina zuordnen kann. Dazu können die den Datenwolken zu Grunde liegenden Volumina des mehrdimensionalen Merkmalsraumes abgesteckt werden, und es wird geprüft, in welches Volumen des Merkmalsraumes die wesentlichen spektralen Merkmale eines neuen Spektrums projiziert werden.

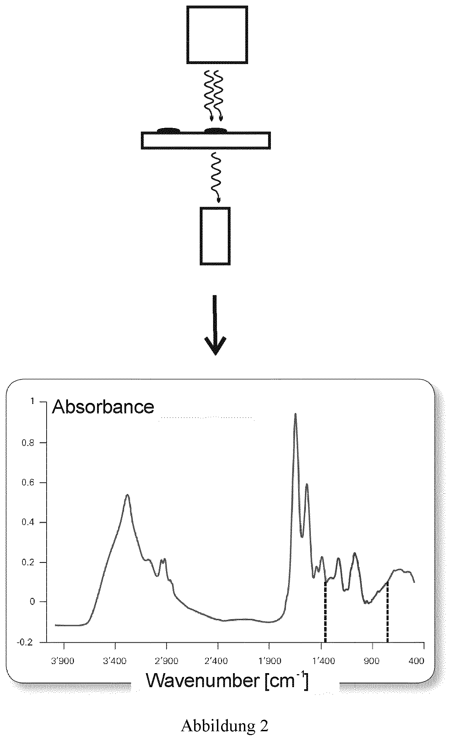

Für eine Infrarot-spektrometrische Charakterisierungsmessung in Transmission kann ein Fourier-Transform-Spektrometer (FT-IR) verwendet werden, das ein hohes Auflösungsvermögen bietet, siehe Messaufbauskizze in Abbildung 2. Typischerweise werden die Spektren von 4000 cm⁻¹ bis zu 500 cm⁻¹ vermessen, wobei der in Abbildung 2 (unten) mit gestrichelter Linie hervorgehobene Bereich zwischen etwa 1350 cm⁻¹ und etwa 700 cm⁻¹ als für die spektrale Unterscheidungskraft besonders ergiebig angesehen wird. Bei Aufnahmeraten von zwanzig Spektren pro Sekunde werden einige Hundert Spektren gemessen und addiert, um das Verhältnis von Signal zu Rauschen zu verbessern.

Die Infrarot-Spektren beruhen auf Tausenden von Vibrationen der funktionalen Gruppen und der polaren Bindungen im biologischen Material; diese wiederum stammen von allen Bestandteilen der Mikroorganismus-Zellen wie DNA, RNA, Proteinen, inneren Strukturen, Membranen, Zellwänden bis hin zu Energiespeichern. Es gibt keine eindeutigen Zuordnungen von Molekülen zu einzelnen Charakteristika in den Spektren, wenn auch bestimmte Spektralbereiche bevorzugt bestimmten Molekülarten zugeordnet werden können: der Fettsäure-Bereich von 3050 bis 2800 cm⁻¹ mit Vibrationen der CH₂- und CH₃-Gruppen, der Amid-Bereich von 1750 bis 1500 cm⁻¹ mit Peptid-Bindungen, der Polysaccharid-Bereich von 1200 bis 900 cm⁻¹. Der Bereich von 900 bis 700 cm⁻¹ wird gelegentlich als Fingerabdruck-Bereich bezeichnet, weil er von allen Molekülen etwas enthält und sehr wichtig für die Unterscheidung der Varietäten ist.

In einer leicht veränderten Ausführungsform können die Infrarot-Spektren auch in reflektiertem Licht gemessen werden. Dazu werden sie auf metallisch spiegelnder Unterlage präpariert, beispielsweise auf Aluminium. Es kann auch die Raman-Spektroskopie zur Anwendung kommen, die den Vorteil hat, die Spektren der präparierten Mikroorganismen auch in Flüssigkeiten messen zu können sowie mit deutlich geringeren Mengen an Probenmaterial auszukommen.

Die aus den Abbildungen 1A und 1B gewonnene Erkenntnis kann für die Erstellung eines varianzkonditionierten Klassifikators in die folgenden Stufen umgesetzt werden:

### (i) Vorbereitung der Referenzmikroorganismen und Festlegung der Varianz(en)

Zunächst werden die zu unterscheidenden Klassen festgelegt. Wird die Kenntnis der Art (Spezies) z.B. von *Streptococcus pneumoniae* als Identität auf der ersten taxonomischen Ebene vorausgesetzt, kann das Ziel die Ermittlung der entsprechenden Serotypen als mögliche Identitäten auf der zweiten untergeordneten taxonomischen Ebene sein. In einem Beispiel können die 23 in klinischen Befunden mit der größten Häufigkeit vorkommenden Serotypen von *Streptococcus pneumoniae* ausgewählt werden. Die Referenzbiomasse dieser Mikroorganismen lässt sich von öffentlich betriebenen Hinterlegungsstellen wie dem Leibniz-Institut DSMZ - Deutsche Sammlung von Mikroorganismen und Zellkulturen GmbH in Braunschweig erhalten.

Um die Organismus-interne Varianz angemessen zu berücksichtigen, kann eine repräsentative Auswahl von Mikroorganismen der zu unterscheidenden Klassen berücksichtigt werden. Je nach Verfügbarkeit können das im Beispiel von *Streptococcus pneumoniae* drei bis sechs verschiedene Stämme pro Serotyp sein; im Fall der 23 häufigsten Serotypen würden so für die Erfassung der Referenzdaten und der Erstellung des Klassifikators 69 bis 138 Stämme in Betracht kommen.

Es wird dann der Parameter festgelegt, dessen Varianz der Erfassung der Referenzdaten aufgeprägt werden soll und dessen variables Auftreten während einer Infrarot-spektrometrischen Messung möglich erscheint. Dies kann eine atmosphärische Varianz sein, z.B. Feuchte, Druck, Gaskonzentration oder Temperatur. Es kann grundsätzlich auch mehr als ein Varianzparameter bei der Erfassung der Referenzdaten berücksichtigt werden, zum Beispiel sowohl Feuchte als auch Temperatur. Allerdings geht eine bezüglich der denkbaren Varianzen breitere Abdeckung auch mit einem entsprechend höheren Messaufwand für die Referenzdaten einher, da die verschiedenen repräsentativen Werte oder Stützwerte der Varianzparameter miteinander kombiniert zu erfassen sind. Es wird eine Liste von Stützpunkten des/der Varianzparameter/s gewählt, die alle realistischen Bedingungen während einer spektrometrischen Messung abdecken. Die Fähigkeit zur Interpolation zwischen den Werten dieser repräsentativen Auswahl an Stützstellen sollte gegeben sein.

### (ii) Erfassen der Referenzdaten

Zunächst lassen sich die Stämme der Referenzmikroorganismen in standardisierter Weise präparieren, also zum Beispiel nach Bebrütung auf oder in einem geeigneten Nährmedium, und ggfs. nach dem Sterilisieren zur Vermeidung biologischer Kontaminationen, auf einem Objektträger für die Infrarot-Spektrometrie in mehreren Replikaten ablegen und dann in den Messschacht einbringen. Der Messschacht wird bezüglich des/der Varianzparameter/s auf einem konstanten vorbestimmten Wert gehalten, beispielsweise 10% relative Feuchte bei 20°C. Nach dem Einbringen des Objektträgers wird bevorzugt eine bestimmte Zeitspanne gewartet, z.B. fünf bis zehn Minuten, damit sich die präparierte Biomasse der Referenzmikroorganismen an die voreingestellten Bedingungen akklimatisieren kann.

Nachdem sich alle Parameter eingeregelt haben, können die Referenzdaten von den präparierten Referenzmikroorganismen bei den voreingestellten Bedingungen erfasst werden. Diese Prozedur wird bei den entsprechend variierten Bedingungen wiederholt, also beispielsweise bei 30%, 55% und 85% relativer Feuchte und konstant 20°C. Jeder Änderung des Varianzwertes sollte eine Akklimatisierungszeitspanne von einigen Minuten folgen, um Einschwingprozesse abklingen zu lassen und reproduzierbar stabile Ergebnisse zu erhalten.

Dieses Verfahren der Referenzdatenerfassung kann durch Messungen nachrangiger Varianzen ergänzt werden, wie sie sich zum Beispiel durch leicht unterschiedliche Bebrütungsbedingungen (biologische Replikate) oder Präparationsbedingungen (z.B. technische Replikate, Verwendung verschiedener Chargen von Re-/Agenzien oder Chemikalien) oder auch durch Messungen auf unterschiedlichen Spektrometern zum Berücksichtigen instrumenteller Varianzen ergeben. Die so erfassten Referenzdaten werden auf Vollständigkeit, offensichtliche Ausreißer (z.B. mittels Methoden des *Local Outlier Factoring,* LOF), und/oder Schlüssigkeit überprüft und gegebenenfalls bereinigt und/oder neu erfasst.

### (iii) Trainieren des varianzkonditionierten Klassifikators

Bevorzugt werden Methoden des maschinellen Lernens verwendet, z.B. Künstliche Neuronale Netze (KNN) oder lineare Diskriminanzanalysen (LDA). Bezüglich der Klassenzugehörigkeit, also z.B. Serotyp #1, Serotyp #2, ..., Serotyp #23 in dem zuvor dargelegten Beispiel *Streptococcus pneumoniae,* ist das Anlernen überwacht (*supervised*). Betreffend der Varianzbedingungen, also verschiedener Umgebungsbedingungen (z.B. Feuchte) oder anderen Einflussfaktoren (z.B. variierende Bebrütung, Präparation, Spektrometer), ist das Anlernen des Klassifikators jedoch nicht überwacht (*unsupervised*). Dies ist gleichbedeutend mit der Maßgabe, die Bedeutung derjenigen spektralen Merkmale in den Referenzdaten, die die Unterscheidungskraft zwischen den einzelnen Klassen (hier Serotypen der Art *Streptococcus pneumoniae*) maximieren, hervorzuheben, wohingegen diejenigen spektralen Merkmale, die stark von den Varianzen beeinflusst sind, geringer gewichtet und damit quasi ausgeblendet werden. Die spektralen Merkmale können sich beispielsweise in den Hauptkomponenten äußern.

Vereinfacht gesagt, und zum Zwecke der Veranschaulichung (ohne Anspruch auf strenge wissenschaftliche Korrektheit), identifiziert der maschinelle Lernalgorithmus diejenigen Teilvolumina in einem üblicherweise mehrdimensionalen multivariaten Merkmalsraum, die jeweils einer der zu unterscheidenden Klassen (d.h. Identitäten auf der zweiten taxonomischen Ebene) zuzuordnen ist. Unerwartet war bei diesem grundsätzlich bekannten Verfahren der Berücksichtigung von Störungen, dass atmosphärische Varianzen wie die relative Feuchte nicht dazu führen, dass die spektralen Merkmale eines Serotyps/Stamms bei variierender Feuchte mit denen von anderen Serotypen/Stämmen überlappen, sondern getrennt bleiben, und somit auch bei solch variierenden Bedingungen Unterscheidbarkeit in einem Raum spektraler Merkmale gewährleisten.

Wie in solchen Anlernphasen unter Verwendung von Referenzdaten üblich lässt sich optional das Leistungsvermögen des resultierenden Klassifikators mittels Kreuzvalidierung testen. Gegebenenfalls kann der maschinelle Lernalgorithmus unter Berücksichtigung der Ergebnisse der Kreuzvalidierung angepasst werden, um die Genauigkeit des Klassifikators weiter zu verbessern.

### (iv) Validierung (optional)

Es kann mit Wissen der taxonomischen Zuordnung eines Prüfmikroorganismus oder mehrerer Prüfmikroorganismen ein Validierungsmesslauf unter Bedingungen durchgeführt werden, die die vorhergesehene Varianz (z.B. variierende relative Feuchte) zulassen, um das Leistungsvermögen auf Grundlage externer Daten zu überprüfen.

Diese Prozedur zur Erstellung eines Klassifikators lässt sich für die Erstellung einer varianzkonditionierten Klassifikator-Datenbank mit den unterschiedlichsten Mikroorganismen, welche wiederum auf unterschiedlichen taxonomischen Ebenen identifiziert sein können, wiederholen. Bevorzugt können Referenzdaten von den im klinischen Umfeld mit der größten Häufigkeit auftretenden Krankheitserregern aufgenommen und verarbeitet werden.

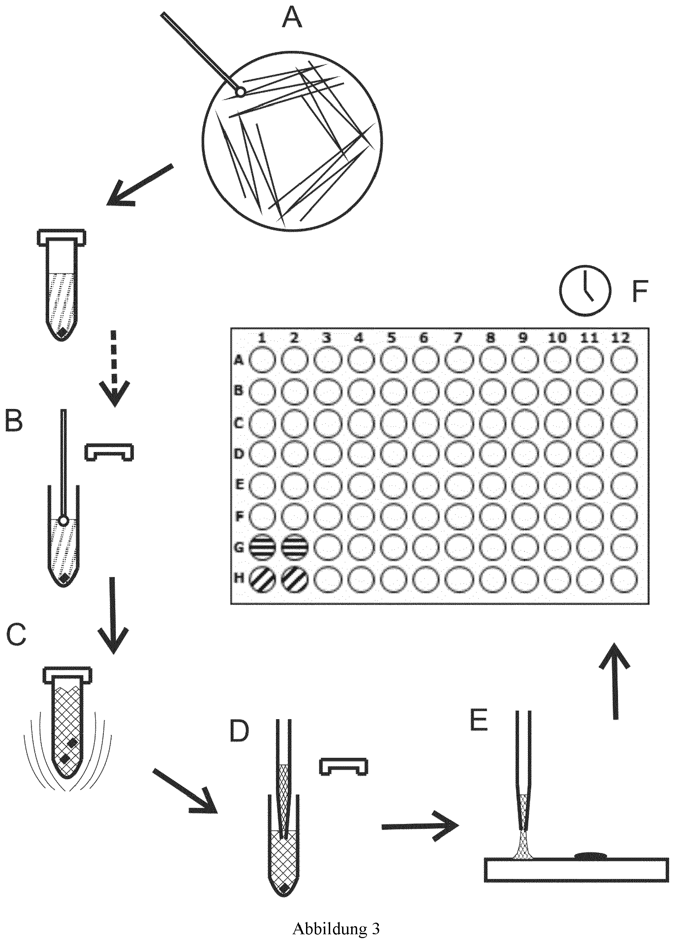

Nachdem der varianzkonditionierte Klassifikator erstellt ist, kann das Verfahren zur Charakterisierung eines Mikroorganismus wie folgt ausgeführt werden, siehe den schematischen Ablauf in Abbildung 3:

Zunächst muss die Identität des Prüfmikroorganismus auf der ersten taxonomischen Ebene bekannt oder ermittelt worden sein, z.B. die Art (Spezies) unter Verwendung eines Massenspektrometers wie dem MALDI Biotyper^{®} (Bruker Daltonik GmbH, Bremen). Davon ausgehend wird der zur ermittelten Identität passende varianzkonditionierte Klassifikator ausgewählt. Beispielhaft sei in diesem Zusammenhang auf das in EP 3 083 981 A1 geschilderte Verfahren verwiesen.

Um ausreichend Biomasse zu erhalten, kann der Prüfmikroorganismus in einer Nährlösung oder auf einem flächigen Nährmedium bebrütet werden. Die so vermehrten Mikroorganismus-Zellen können dann dem Nährmedium entnommen werden, beispielsweise durch Abscheiden aus der Nährlösung, z.B. über Zentrifugieren oder Filtern, oder durch Beproben von einer Agarplatte. Zwecks Sterilisierung lassen sich die derart geernteten Mikroorganismen in einer aktivitätshemmenden Flüssigkeit wie Ethanol (z.B. 70% v/v) resuspendieren.

Mikroorganismen reagieren sehr empfindlich auf Veränderungen der Wachstumsbedingungen wie unterschiedliche Medien, Temperaturen, Nährstoffe, Veränderungen der Gasversorgung (Sauerstoff und andere), Feuchtigkeit, Bebrütungsdauer usw. Diese Faktoren können Veränderungen in der Zellzusammensetzung und im Stoffwechsel hervorrufen, die mit der Infrarot-Spektrometrie erkannt werden können. Zur Bebrütung kann das Zellmaterial einer reinen Einzelkolonie mit einem Spatel auf einer Agarplatte ausgebreitet werden, um konfluentes Wachstum hervorzurufen. Diese Technik ermöglicht die Sammlung von Zellen in einer sehr reproduzierbaren Mischung der verschiedenen Wachstumsphasen, die in Kolonien immer vorhanden sind. Für die meisten klinisch relevanten Stämme beträgt die optimale Inkubationszeit etwa 16 bis 24 Stunden, und die häufig für Bakterien anwendbare Bebrütungstemperatur liegt bei etwa 35 bis 37°C. Das Probenmaterial eines bebrüteten Prüfmikroorganismus kann direkt aus der Mitte des Zellrasens z.B. unter Verwendung einer kalibrierten Platinöse mit einem Durchmesser von einem Millimeter geerntet werden (Schritt A).

Von einem flächigen Nährmedium wie Agar lässt sich Biomasse des vermehrten Prüfmikroorganismus aus einer oder mehreren Kolonien beproben und unmittelbar auf einem spektrometrischen Objektträger ablegen, wobei auf gleichmäßige Verteilung zu achten ist, und optional durch Bestrahlung mit ultraviolettem Licht sterilisieren (z.B. bei *Streptococcus pneumoniae*). Alternativ kann die Biomasse ebenfalls in eine Stoffwechsel-hemmende Flüssigkeit resuspendiert werden (Schritt B). Die Flüssigkeit kann auch de-ionisiertes Wasser sein, das üblicherweise keine Stoffwechsel-hemmende Wirkung ausübt. Auch in diesem Fall lässt sich der Prüfmikroorganismus nach dem Ablegen auf einer Probenstelle eines Objektträgers durch UV-Bestrahlung oder anderweitige Energieeinwirkung (z.B. Hitze) sterilisieren.

Zu beachten ist, dass dem aus oder von dem Nährmedium entnommenen Prüfmikroorganismus keine Reste des Nährmediums anhaften, die das Messergebnis stören könnten. Um eine gleichmäßige Verteilung der Biomasse des Prüfmikroorganismus in der Suspension zu erhalten, können kleine Zylinder oder Kügelchen aus reaktionsinertem Material wie Stahl der Suspension beigemengt und das verschlossene Suspensionsgefäß dann geschüttelt werden (Schritt C). Die Suspension wird dann aliquotiert und in Replikaten (Schritt D), deren Anzahl von Protokoll zu Protokoll variieren kann, sachte auf den Objektträger gebracht, z.B. unter Verwendung einer Pipette mit Plastikspitze, wobei eine gleichmäßige Auftragung mit homogener Schichtdicke die besten Messergebnisse verspricht (Schritt E). Nach dem Aufbringen aller zu untersuchenden Proben auf den Objektträger lässt man diesen zum Trocknen der Suspensionen für einige Minuten, z.B. zehn bis dreißig Minuten, bei einer festgelegten Temperatur, z.B. 37°C, stehen (Schritt F). Wird der Prüfmikroorganismus nach der Ernte aus dem Bebrütungsgefäß unmittelbar ohne weiteres Resuspendieren auf den Objektträger aufgebracht, kann das Trocknen entfallen oder zumindest deutlich kürzer ausfallen.

Die so präparierten Objektträger können dann in einen Messschacht eines Spektrometers eingeführt und Probe für Probe unter Bedingungen, die den Einfluss wenigstens einer Varianzquelle zulassen, gemessen werden. Einige Stellen auf dem Objektträger können auch mit Teststandard-Biomasse belegt werden, um die technische Leistungsfähigkeit des Spektrometers zu überprüfen, beispielsweise angelehnt an das in EP 3 392 342 A1 erläuterte Verfahren der Anmelderin.

Die erfassten Spektren können nach den üblichen Prozessierschritten wie Grundliniensubtraktion, Glättung und Bildung der zweiten Ableitung einer Analyse mit dem zuvor erstellten varianzkonditionierten Klassifikator unterworfen werden. Wie zuvor beschrieben werden dabei nur (zumindest aber vorherrschend) diejenigen spektralen Merkmale berücksichtigt, die von der Varianz gar nicht oder nur wenig beeinflusst werden, wohingegen solche spektralen Merkmale, die eine hohe Varianz-bedingte Variation aufweisen, weitgehend oder vollständig ausgeblendet werden.

Die Verarbeitung der Messdaten mit dem varianzkonditionierten Klassifikator führt zu einer Zuordnung des untersuchten Spektrums zu einer der möglichen Identitäten auf der zweiten taxonomischen Ebene, im Beispiel des *Streptococcus pneumoniae* einem der referenzierten Serotypen. Nur in seltenen Fällen gelingt keine verlässliche Charakterisierung, beispielsweise auf Grund unvorhergesehener Störungen bei der Bebrütung, Probenpräparation oder Messung oder weil die gesuchte Identität des Prüfmikroorganismus auf der zweiten taxonomischen Ebene in den Referenzdaten nicht berücksichtigt ist (z.B. im Fall eines sehr seltenen, für die klinische Praxis nahezu irrelevanten Serotyps).

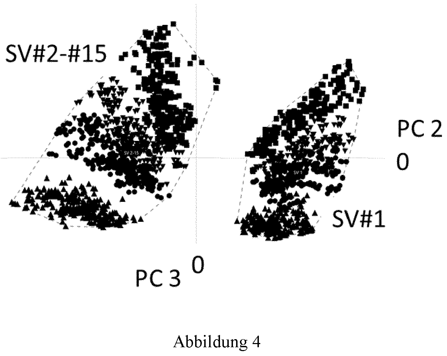

Abbildung 4 zeigt ein weiteres Beispiel der fortgesetzten Unterscheidbarkeit von Serotypen auch unter variierenden Feuchteniveaus in einem Hauptkomponentenraum, der hier der Übersichtlichkeit halber zweidimensional gezeigt ist (PC 2, PC 3). Grundlage ist die Art *Legionella pneumophila,* von der fünfzehn Serotypen in der Darstellung spektraler Merkmale berücksichtigt sind. Allerdings sind die Serotypen nicht einzeln klassifiziert, sondern in stark pathogene und weniger pathogene zusammengefasst. Dies unterscheidet den ersten Serotyp SV#1 (rechte Datenwolke), der für 70 bis 80 Prozent aller Legionellosen beim Menschen verantwortlich ist, von den nächsten vierzehn SV#2 bis SV#15, die deutlich weniger pathogen sind, wenn überhaupt. In ähnlicher Weise könnten auch suszeptible (empfindliche) und resistente Serotypen/Stämme zusammen in Gruppen klassifiziert werden.

Die Referenzdaten der verschiedenen Serotypen der Referenzmikroorganismen *Legionella pneumophila,* deren zu Grunde liegende Stämme mit unterschiedlichen Symbolen wie Dreiecken, Quadraten und Kreisen kodiert sind, wurden unter vier verschiedenen relativen Feuchten aufgenommen (arid 10%, semi-arid 30%, feucht 55% und tropisch 85%). In der Darstellung äußert sich diese Varianz im Wesentlichen in der Erstreckung der Datenwolken entlang der Hauptkomponentenachse PC 2. Deutlich ist jedoch, dass die zum Serotyp 1 (SV#1) gehörende Datenwolke, ungeachtet der Varianz, hinreichend von der zusammengefassten Datenwolke der anderen Serotypen SV#2 bis SV#15 entfernt ist, um die Unterscheidbarkeit auf der Grundlage spektraler Merkmale zu gewährleisten. Sollten sich in einem Klinikum Durchfallerkrankungen häufen, die sich in einer ersten Analyse, beispielsweise mit der etablierten massenspektrometrischen Methode MALDI-TOF, der Bakterienart *Legionella pneumophila* zuschreiben lassen, kann bei der weiteren Infrarot-spektrometrischen Untersuchung des isolierten und bebrüteten Krankheitserregers ein entsprechend trainierter Klassifikator zur Unterscheidung des besonders pathogenen Serotyps SV#1 von den anderen weniger gefährlichen Serotypen SV#2 bis SV#15 eingesetzt werden, um im Fall eines positiven Befunds eine gezielte Behandlung beginnen zu können. Diese Vorgehensweise lässt sich natürlich auf andere Mikroorganismen übertragen. Der Flexibilität der hier beschriebenen Klassifikator-Erstellung sind keine Grenzen gesetzt.

Ausgehend von den zuvor beschriebenen Methoden lassen sich varianzkonditionierte Klassifikatoren für eine Vielzahl möglicher Mikroorganismen und ebenso für eine Vielzahl möglicher Varianzquellen, einzeln oder auch mehrere kombiniert, während einer spektrometrischen Messung ermitteln. Auf diese Weise kann in Kenntnis der Identität eines zu charakterisierenden Mikroorganismus auf einer ersten taxonomischen Ebene durch Wahl des passenden varianzkonditionierten Klassifikators eine spektrometrische Subcharakterisierung der Identität auf einer zweiten nachgeordneten taxonomischen Ebene robust und verlässlich erfolgen.

Neben den beispielhaft erläuterten Ausführungen sind noch weitere Ausführungsformen der Erfindung denkbar. Der Gegenstand, für den Schutz begehrt wird, ist in den angehängten Patentansprüchen definiert. In Kenntnis dieser Offenbarung ist es dem Fachmann ohne weiteres möglich, weitere vorteilhafte Ausführungsformen zu entwerfen, die vom Schutzbereich der angehängten Patentansprüche umfasst sind.

## Patentansprüche

1. Verfahren zum spektrometrischen Charakterisieren von Mikroorganismen, aufweisend:
- Bereitstellen eines Prüfmikroorganismus, dessen Identität auf einer ersten taxonomischen Ebene bekannt ist;
- Sterilisieren des Prüfmikroorganismus;
- Aufnehmen spektrometrischer Messdaten vom sterilisierten Prüfmikroorganismus unter Bedingungen, die den Einfluss wenigstens einer Varianzquelle zulassen, welche nicht in der taxonomischen Einordnung des Prüfmikroorganismus begründet ist;
- Auswählen eines Klassifikators, der darauf trainiert ist, die Identität eines Mikroorganismus auf einer zweiten taxonomischen Ebene, die der ersten taxonomischen Ebene nachgeordnet ist, zu ermitteln, wobei mögliche Identitäten des Klassifikators auf der zweiten taxonomischen Ebene der bekannten Identität des Prüfmikroorganismus auf der ersten taxonomischen Ebene zugeordnet sind; und
- Anwenden des Klassifikators auf die Messdaten, um die Identität des Prüfmikroorganismus auf der zweiten taxonomischen Ebene zu ermitteln;
- wobei der Klassifikator varianzkonditioniert ist, indem er erhalten ist durch Trainieren auf gezielt varianzbehafteten spektrometrischen Referenzdaten verschiedener bekannter Referenzmikroorganismen, welche die gleiche Identität auf der ersten taxonomischen Ebene wie der Prüfmikroorganismus aufweisen und verschiedene Identitäten auf der zweiten taxonomischen Ebene abdecken, wobei das Trainieren die Maßgabe umfasst, diejenigen spektralen Merkmale eines ersten Typs aus den Referenzdaten, die eine Unterscheidung der verschiedenen Identitäten auf der zweiten taxonomischen Ebene fördern, gegenüber denjenigen spektralen Merkmalen eines zweiten Typs aus den Referenzdaten, die von der gezielten Varianz beeinflusst sind, aufzugewichten, um den Varianzeinfluss bei der Charakterisierung des Prüfmikroorganismus auf der zweiten taxonomischen Ebene weitgehend oder vollständig auszublenden.

2. Verfahren nach Anspruch 1, bei dem das Bereitstellen die Isolierung des Prüfmikroorganismus aus einem Habitat umfasst.

3. Verfahren nach Anspruch 2, bei dem das Habitat eine biologische und/oder chemische Matrix ist.

4. Verfahren nach Anspruch 3, bei dem das Isolieren des Prüfmikroorganismus das Entfernen der Matrix beinhaltet.

5. Verfahren nach einem der Ansprüche 1 bis 4, bei dem das Bereitstellen des Prüfmikroorganismus einen Vermehrungsschritt beinhaltet.

6. Verfahren nach einem der Ansprüche 1 bis 5, bei dem das Sterilisieren ein Exponieren des Prüfmikroorganismus gegenüber einer Stoffwechsel-hemmenden Flüssigkeit oder Energieeinwirkung umfasst.

7. Verfahren nach einem der Ansprüche 1 bis 6, bei dem die erste taxonomische Ebene und die zweite taxonomische Ebene unmittelbar benachbart sind.

8. Verfahren nach Anspruch 7, bei dem die erste taxonomische Ebene der Art/Spezies und die zweite taxonomische Ebene der Unterart/Subspezies entspricht.

9. Verfahren nach Anspruch 7 oder Anspruch 8, bei dem die erste taxonomische Ebene der Art/Spezies entspricht und die zweite taxonomische Ebene verschiedene Varietäten umfasst, z.B. pathogene und nicht-pathogene Varietäten, resistente und empfindliche/ suszeptible Varietäten oder verschiedene Stämme der Art.

10. Verfahren nach einem der Ansprüche 1 bis 9, bei dem die Identität des Prüfmikroorganismus auf der ersten taxonomischen Ebene vorab durch wenigstens eines der folgenden Verfahren ermittelt wurde: (i) Massenspektrometrie, (ii) Infrarot-Spektrometrie, (iii) Wachstum auf selektiven Medien, was beispielsweise unter den Begriff "bunte Reihe" fällt, und (iv) Gensequenzanalysen.

11. Verfahren nach einem der Ansprüche 1 bis 10, bei dem die wenigstens eine Varianz atmosphärischen Ursprungs ist.

12. Verfahren nach Anspruch 11, bei dem die wenigstens eine Varianz unterschiedliche Werte auf wenigstens einer der folgenden Skalen umfasst: Temperatur, Luftfeuchte, Druck und Kohlendioxid-Gehalt der Umgebungsluft.

13. Verfahren nach einem der Ansprüche 1 bis 12, bei dem der Klassifikator mit Hilfe von Methoden des maschinellen Lernens, z.B. künstlicher neuronaler Netze, "KNN", oder linearer Diskriminanzanalysen, "LDA", erhalten und trainiert wird.

14. Verfahren nach einem der Ansprüche 1 bis 13, bei dem die Charakterisierung Infrarot-spektrometrische Methoden verwendet.

15. Verfahren nach einem der Ansprüche 1 bis 14, bei dem das Sterilisieren ein Exponieren des Prüfmikroorganismus gegenüber einer Stoffwechsel-hemmenden Flüssigkeit und Energieeinwirkung auf den Prüfmikroorganismus umfasst.

## Claims

1. A method for the spectrometric characterization of microorganisms, comprising:
- Providing a test microorganism whose identity is known on a first taxonomic level;
- Sterilizing the test microorganism;
- Acquiring spectrometric measurement data from the sterilized test microorganism under conditions which allow the influence of at least one source of variance that is not based on the taxonomic classification of the test microorganism;
- Selecting a classifier which is trained to determine the identity of a microorganism on a second taxonomic level which is subordinate to the first taxonomic level, where possible identities of the classifier on the second taxonomic level are assigned to the known identity of the test microorganism on the first taxonomic level, and
- Applying the classifier to the measurement data in order to determine the identity of the test microorganism on the second taxonomic level;
- wherein the classifier is variance-conditioned by obtaining it through training on target-edly variance-loaded spectrometric reference data of different known reference microorganisms which exhibit the same identity as the test microorganism on the first taxonomic level and cover different identities on the second taxonomic level, where the training includes the stipulation of giving greater weighting to those spectral characteristics of a first type from the reference data which promote the differentiation of the different identities on the second taxonomic level, than to those spectral characteristics of a second type from the reference data which are affected by the targeted variance, in order to largely or completely mask out the effect of variance in the characterization of the test microorganism on the second taxonomic level.

2. The method according to Claim 1, wherein the providing includes the isolation of the test microorganism from a habitat.

3. The method according to Claim 2, wherein the habitat is a biological and/or chemical matrix.

4. The method according to Claim 3, wherein the isolation of the test microorganism includes the removal of the matrix.

5. The method according to any one of Claims 1 to 4, wherein the providing of the test microorganism includes a multiplication step.

6. The method according to any one of Claims 1 to 5, wherein the sterilizing includes the exposure of the test microorganism to a metabolism-inhibiting liquid or to an impact of energy.

7. The method according to any one of Claims 1 to 6, wherein the first taxonomic level and the second taxonomic level are immediately adjacent to each other.

8. The method according to Claim 7, wherein the first taxonomic level corresponds to the species and the second taxonomic level corresponds to the subspecies.

9. The method according to Claim 7 or Claim 8, wherein the first taxonomic level corresponds to the species and the second taxonomic level comprises different varieties, e.g., pathogenic and non-pathogenic varieties, resistant and susceptible varieties, or different strains of the species.

10. The method according to any one of Claims 1 to 9, wherein the identity of the test microorganism on the first taxonomic level was determined in advance by means of at least one of the following procedures: (i) mass spectrometry, (ii) infrared spectrometry, (iii) growth on selective media, which by way of example falls within the ambit of the term "colorful series", and (iv) gene sequence analyses.

11. The method according to any one of Claims 1 to 10, wherein the at least one variance is of atmospheric origin.

12. The method according to Claim 11, wherein the at least one variance has different values on at least one of the following scales: temperature, humidity, pressure, and carbon dioxide content of the ambient air.

13. The method according to any one of Claims 1 to 12, wherein the classifier is obtained and trained with the aid of procedures of machine learning, e.g., artificial neural networks, "ANN", or linear discriminant analyses, "LDA".

14. The method according to any one of Claims 1 to 13, wherein the characterization uses infrared spectrometric procedures.

15. The method according to any one of Claims 1 to 14, wherein the sterilizing includes the exposure of the test microorganism to a metabolism-inhibiting liquid and to an impact of energy on the test microorganism.

## Revendications

1. Méthode de caractérisation spectrométrique des micro-organismes, comprenant :
- La fourniture d'un micro-organisme test dont l'identité est connue à un premier niveau taxonomique ;
- La stérilisation du micro-organisme test ;
- L'acquisition de données de mesure spectrométriques à partir du micro-organisme test stérilisé dans des conditions qui permettent l'influence d'au moins une source de variance qui n'est pas basée sur la classification taxonomique du micro-organisme test ;
- La sélection d'un classificateur entraîné à déterminer l'identité d'un micro-organisme à un second niveau taxonomique subordonné au premier niveau taxonomique, les identités possibles du classificateur au second niveau taxonomique étant attribuées à l'identité connue du micro-organisme test au premier niveau taxonomique, et
- L'application du classificateur aux données de mesure afin de déterminer l'identité du micro-organisme test au second niveau taxonomique ;
- dans laquelle le classificateur est conditionné par la variance en l'obtenant par entraînement sur des données de référence spectrométriques ciblément chargées de variance de différents micro-organismes de référence connus qui présentent la même identité que le micro-organisme test au premier niveau taxonomique et couvrent des identités différentes au second niveau taxonomique, l'entraînement comprend à donner plus de poids aux caractéristiques spectrales d'un premier type des données de référence qui favorisent la différenciation des différentes identités au second niveau taxonomique, qu'aux caractéristiques spectrales d'un deuxième type des données de référence qui sont affectées par la variance ciblée, afin de masquer largement ou complètement l'effet de la variance dans la caractérisation du micro-organisme test au second niveau taxonomique.

2. Méthode selon la revendication 1, dans laquelle la fourniture comprend l'isolement du micro-organisme test à partir d'un habitat.

3. Méthode selon la revendication 2, dans laquelle l'habitat est une matrice biologique et/ou chimique.

4. Méthode selon la revendication 3, dans laquelle l'isolement du micro-organisme test comprend l'élimination de la matrice.

5. Méthode selon l'une quelconque des revendications 1 à 4, dans laquelle la fourniture du micro-organisme test comprend une étape de multiplication.

6. Méthode selon l'une quelconque des revendications 1 à 5, dans laquelle la stérilisation comprend l'exposition du micro-organisme test à un liquide inhibant le métabolisme ou à un impact d'énergie.

7. Méthode selon l'une quelconque des revendications 1 à 6, dans laquelle le premier niveau taxonomique et le second niveau taxonomique sont immédiatement adjacents l'un à l'autre.

8. Méthode selon la revendication 7, dans laquelle le premier niveau taxonomique correspond à l'espèce et le second niveau taxonomique correspond à la sous-espèce.

9. Méthode selon la revendication 7 ou la revendication 8, dans laquelle le premier niveau taxonomique correspond à l'espèce et le second niveau taxonomique comprend différentes variétés, par exemple des variétés pathogènes et non pathogènes, des variétés résistantes et sensibles, ou différentes souches de l'espèce.

10. Méthode selon l'une quelconque des revendications 1 à 9, dans laquelle l'identité du micro-organisme test au premier niveau taxonomique a été déterminée au préalable au moyen d'au moins un des procédés suivants : (i) spectrométrie de masse, (ii) spectrométrie infrarouge, (iii) croissance sur des milieux sélectifs, ce qui, à titre d'exemple, relève de l'expression "série colorée", et (iv) analyses de séquences génétiques.

11. Méthode selon l'une quelconque des revendications 1 à 10, dans laquelle l'au moins une variance est d'origine atmosphérique.

12. Méthode selon la revendication 11, dans laquelle l'au moins une variance a des valeurs différentes sur au moins une des échelles suivantes : température, humidité, pression et teneur en dioxyde de carbone de l'air ambiant.

13. Méthode selon l'une quelconque des revendications 1 à 12, dans laquelle le classificateur est obtenu et entraîné à l'aide de procédés d'apprentissage automatique, par exemple les réseaux neuronaux artificiels, "artificiel neural networks, ANN", ou les analyses discriminantes linéaires, "linear discriminant analyses, LDA".

14. Méthode selon l'une quelconque des revendications 1 à 13, dans laquelle la caractérisation utilise des procédés spectrométriques infrarouges.

15. Méthode selon l'une quelconque des revendications 1 à 14, dans laquelle la stérilisation comprend l'exposition du micro-organisme test à un liquide inhibant le métabolisme et à un impact d'énergie sur le micro-organisme test.
